Europäisches Patentamt

European Patent Office

Office européen des brevets

(11) Publication number: **0 272 467 B1**

# EUROPEAN PATENT SPECIFICATION

(45) Date of publication of patent specification: **22.01.92**  (51) Int. Cl.⁵: **A61M 5/168**

(21) Application number: **87117239.1**

(22) Date of filing: **23.11.87**

(54) **A device for controlling liquid dripping.**

(30) Priority: **18.12.86 JP 299944/86**
**18.12.86 JP 193759/86 U**

(43) Date of publication of application:
**29.06.88 Bulletin  88/26**

(45) Publication of the grant of the patent:
**22.01.92 Bulletin  92/04**

(84) Designated Contracting States:
**BE DE ES FR LU NL**

(56) References cited:
**DE-A- 2 917 270**
**US-A- 3 601 124**
**US-A- 4 452 273**

(73) Proprietor: **KAWASUMI LABORATORIES, INC.**
**No. 28-15, Minami-Ohi, 3-chome Shinagawa-ku**
**Tokyo(JP)**

(72) Inventor: **Sugisaki, Yuzuru**
**No. 840, Hirai Kannamicho**
**Tagata-gun Shizuoka-ken(JP)**
Inventor: **Takada, Makoto**
**No. 552-1, Gotenba**
**Gotenba-shi Shizuoka-ken(JP)**
Inventor: **Tokuda, Kazuhiko**
**No. 751-5, Mototachino Shuzenji-cho**
**Tagata-gun Shizuoka-ken(JP)**
Inventor: **Suzuki, Yasuo**
**No. 18, Utsukushigaoka, 1-chome Midori-ku**
**Yokohama-shi Kanagawa-ken(JP)**
Inventor: **Oya, Takashi**
**No. 23-9, Miyashita-Honcho, 1-chome**
**Sagamihara-shi Kanagawa-ken(JP)**
Inventor: **Tomeba, Shoichi**
**No. 15-7, Yokoyamadai, 1-chome**
**Sagamihara-shi Kanagawa-ken(JP)**
Inventor: **Yoshida, Shinichiro**
**No. 11-5, Hishigaoka, 4-chome**
**Sagamihara-shi Kanagawa-ken(JP)**

(74) Representative: **von Bezold, Dieter, Dr. et al**
**Dr. Dieter von Bezold Dipl.-Ing. Peter Schütz**
**Dipl.-Ing. Wolfgang Heusler Brienner Strasse 52**
**W-8000 München 2(DE)**

# Description

The present invention relates to a device for controlling the dripping rate of a liquid, comprising a flexible tube, a stationary clamping jaw and a movable clamping jaw between which said flexible tube is positioned for controlling the amount of throttling of said tube by the movable clamping jaw so as to adjust the liquid dripping rate.

When a medical liquid contained in a container is injected into the interior of a patient's body through a liquid sending set, the dripping rate of the medical liquid must be controlled appropriately.

Conventionally a manual roll clamp has been used as dripping rate controlling device. However, since the dripping rate changes as time passes and the roll has to be adjusted from time to time, a device which automatically controls the dripping rate has been recently become known.

Fig. 8 is a schematic view of a known liquid sending or perfusion control device . The numeral 100 is a container holding the liquid, and 101 is a liquid carrying set for introducing the medical liquid into the interior of the patient's body. The liquid sending set 101 comprises a cannula or liquid needle 102 to be pierced into a mouth part of the container, a dripping tube 104, flexible tubes 103, 105 to be connected to the upper and lower parts of the dripping tube 104, and an injection needle (not shown) to be connected to the lower tube 105.

A liquid carrying control device 115 is composed of a dripping sensor 106 to be attached to the dripping tube 104 and a control device 107.

The control device 107 is, as shown in Fig. 9, formed with a tube holding groove 110 centrally in a vertical direction, and provided with a stationary clamping jaw 108 and a movable clamping jaw 109 in opposition each other at a lower part of the tube holding groove 110.

The numeral 111 is a dripping number or rate set switch, 112 is an electric power switch, 113 is an alarm lamp, and 114 is a thumb for opening or closing the movable clamping jaw 109 when the tube 105 is attached into or detached from the groove 110.

This known device detects the number of the drops of the medical liquid in the dripping tube 104 by means of the dripping sensor 106, calculates therefrom the dripping rate (number of drops per unit of time) by a calculation device installed in the control device 107, moves the clamping jaw 109 at constant speed so as to provide a predetermined dripping rate, and automatically controls the amount of throttling of the tube 105.

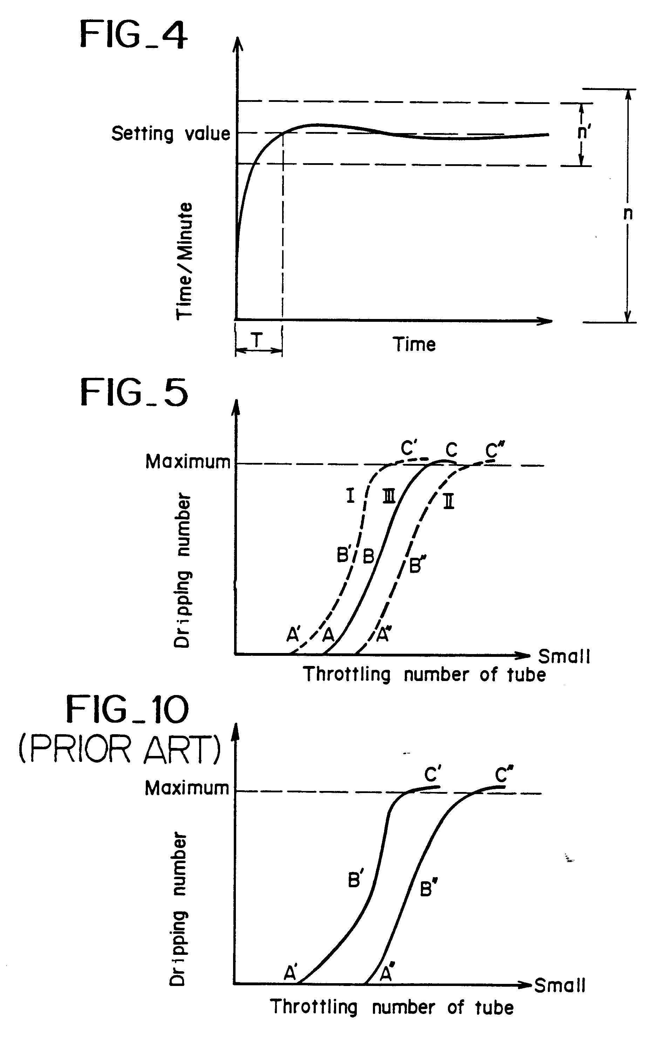

However, since the liquid carrying control device only moves the clamping jaw 109 at a constant speed for controlling the amount of throttling of the tube 105, it is difficult to bring the dripping rate to a predetermined value rapidly and stably when starting to send the liquid, besides resilience and recovery of the flexible tube 105. This will be explained with reference to Fig. 10.

Fig. 10 is a diagram showing the relationship between the amount of throttling of the flexible tube 105 and the liquid dripping rate, where A′- B′ - C′ and A″ - B″ - C″ are S-shaped. curves proper to certain two kinds of liquid sending sets, and A′ and A″ are the points where the 1st drops appear, and B′ and B″ are approximately around the predetermined values, and C′ and C″ are the points where the maximum driping rate is assumed.

As is apparent from the diagram, with respect to the flexible tube 105 held between the stationary clamping jaw 108 and the movable clamping jaw 109, its opening area is not proportional to the liquid flowing rate, and the number of drops is rapidly changed around A′, A″ and B′, B″ by slightly changing the amount of throttling. Therefore, when the clamping jaw 109 is moved at a certain speed, it is difficult to bring the throttling amount closely to the predetermined value.

For example, if the movable clamping jaw 109 is moved at high speed for starting to send the liquid earlier, said changing of the dropping number exceeds a large range, and when the movable clamping jaw 109 is returned at high speed for revising said exceeding, this returning would overrun.

If the clamping jaw 109 is moved at the predetermined high speed, it is difficult to converge the dropping number closely to the predetermined valus B′, B″, and the dripping rate becomes unstable.

If the clamping jaw 109 is moved with a predetermined low speed for cancelling unstability, it takes much time (starting time of sending the liquid) to move the clamping jaw 109 from a zero position (the position where the tube is completely closed) to the position at which the dripping starts.

Further, if the clamping jaw 109 is moved at a determined middle speed, there occurs a defect involved with any one of the high speed movement and the low speed movement.

On the other hand, when the flexible tube 105 is attached to the control device 107 and since the tube 105 is inserted successively in the length of the narrow groove 110, the attachment is troublesome. Besides, the tube 105 is pressed in the groove 110 under pressure, so that the tube is loaded over a full length, and a part of half way is twisted or caused with strain, and the medical liquid does not flow smoothly.

From US-A-3 601 124 is known a device for controlling the dripping rate of a medical liquid for blood transfusions, intravenous feeding and other applications, wherein a clamp is operated by a motor to either constrict or open a flexible tube in response to a reference signal which is directly proportional to the rate at which drips are allowed to fall through a drip chamber and a liquid drip signal which is directly proportional to the number of sensed drips. For controlling the dripping rate of the liquid, the reference signal acts to drive the motor in a direction to release the tube, while detection of a drip acts to drive the motor in the direction to constrict the tube. This results in an oscillating motion and when the control system is balanced, the clamp oscillates back and forth about an an average clamping value.

A similar device for controlling the dripping rate of a medical liquid is disclosed in DE-A-2 917 270, wherein the dripping rate in a similar way is controlled by a closed feedback loop. This known device further includes means for stopping and/or providing an alarm signal when an abnormal dripping rate occurs.

It is an object of the invention to provide a device which may control the movable clamping jaw, where the throttling amount of the flexible tube is changed by moving the clamping jaw so as to control the liquid dripping rate by starting the liquid dripping in a short time, and after then the dripping rate is brought gradually to the determined value, so that the dripping rate is made stable.

It is another object of the invention to provide a device which is of high safety, where the movable clamping jaw exactly closes the flexible tube in an abnormal case of the dripping rate so as not to inject an excessive amount of liquid into the interior of the body.

It is a further object of the invention to provide a device which is of high efficiency and high safety, where a microcomputer is employed to simplify a control part and a mechanism part.

It is still further object of the invention to provide an improved device so that the flexible tube may be easily attached thereto without causing twisting, strain or loading, and the liquid within the tube flows smoothly.

The above mentioned objects may be accomplished by the device as defined in the claims.

According to the invention, the device for controlling a liquid dripping rate of a medical liquid spending set which has a flexible tube for leading said liquid, and a dripping tube connected to said flexible tube, comprises

a stationary clamping jaw and a movable clamping jaw for clamping said flexible tube,

a device for detecting dripping of said liquid into said dripping tube,

control means for controlling operation of a drive device of said movable clamping jaw in association with said drip detecting device,

said control means being provided with means for feedback controlling the dripping rate to be a determined rate while moving at a first speed said movable clamping jaw in the direction of throttling or releasing said flexible tube, and is

characterized in that said control means further is provided with

means for moving said movable clamping jaw in a direction of throttling said flexible tube by a signal for starting the control, and completely closing said flexible tube by said stationary and said movable clamping jaws,

means for moving at a second speed said drive device of said movable clamping in the direction of releasing said flexible tube, said second speed being higher than said first speed, so as to rapidly start dripping from the closed condition of said flexible tube,

throttling the tube and stopping said movable clamping jaw; and

means for causing the microcomputer to issue a low speed control signal from a third drip to a n-th drip, and moving the movable clamping jaw to a direction of releasing the tube at said first speed.

An embodiment of the invention will be explained with reference to the attached drawings.

Fig. 1     is a block diagram exemplifying a liquid dripping control device according to the invention;

Fig. 2     is a flow chart showing a method of controlling the liquid dripping by the invention;

Fig. 3     is a block diagram explaining actuation of a microcomputer of the invention;

Fig. 4     is a diagram showing changes of the dropping number as time passes in the invention;

Fig. 5     is a diagram showing relationship between the drop number and the throttling amount of the tube by the invention;

Fig. 6     is a front view of a control device of the invention;

Fig. 7     is a side view of the above control device;

Fig. 8     is an outlined view showing use of a conventional liquid dripping control device;

Fig. 9     is a front view of the above conventional device; and

Fig. 10    is a diagram showing relation between the throttling amount of an ordinary bendable tube and the dropping number.

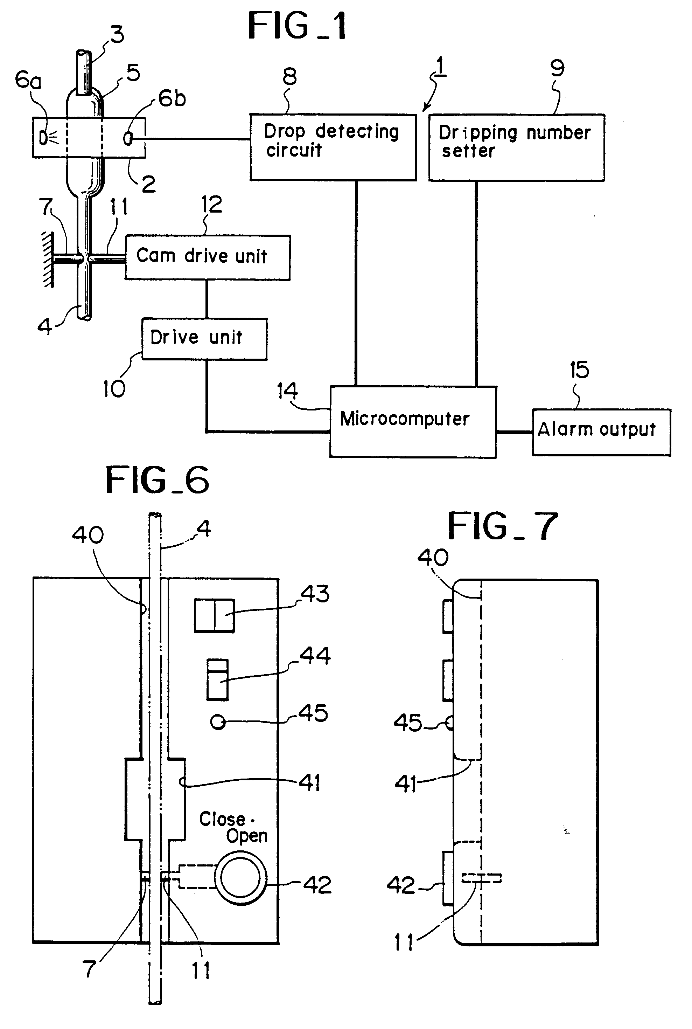

In Fig. 1, the numeral 5 designates a dripping tube which constitutes one part of a liquid sending set or perfusion device. 3 is a flexible tube connected to an upper part of the dripping tube 5. 4 is also a flexible tube connected to the lower part thereof. Although not shown, the upper tube 3 is connected to a liquid needle or cannula to be pierced into a mouth part of a medical liquid container, and the lower tube 4 is connected with an injection needle to be pierced into a patient at a blood vessel. The dripping tube 5 and the tubes 3, 4 are made of transparent or half transparent vinyl chloride.

The present invention is a device which controls the dripping rate of the medical liquid flowing in the liquid sending set, an entire body of which is shown with the numeral 1.

The numeral 2 designates a liquid dripping sensor to be detachably attached to the dripping tube 5 where a light emitting diode 6a and a light receiving or photo diode 6b are installed. When the liquid drips into the dripping tube 5 through the upper tube 3, the dripping liquid interrupts the light path between the diodes 6a, 6b and a liquid drop detecting circuit 8 then detects a change of light amount, and a signal is input into a microcomputer 14.

The numeral 7 is a stationary clamping jaw and 11 is an opposed movable clamping jaw and the lower flexible tube 4 is held by these clamping jaws (which may also be denoted "klemmen"). If the clamping jaw 11 is moved to change the amount of throttling of the tube 4, the dripping rate of the liquid in the dripping tube 5 is adjusted.

12 is a cam drive unit for moving the clamping jaw 11, which comprises a drive motor, cams and gears.

10 is a drive unit which responds to a signal from a microcomputer 14, and sends the signal to the drive motor of the clamping jaw 11, and controls the rotation speed and direction of the drive motor.

9 is a dripping number setter which inputs a required dripping speed to the microcomputer 14.

15 is an alarm output which detects abnormal dripping speed and other troubles, or sends the alarm output signal to the drive circuit 10 of the cam drive unit 12, so that the clamping jaw 11 is moved at high speed in the throttling direction to stop sending of the medical liquid.

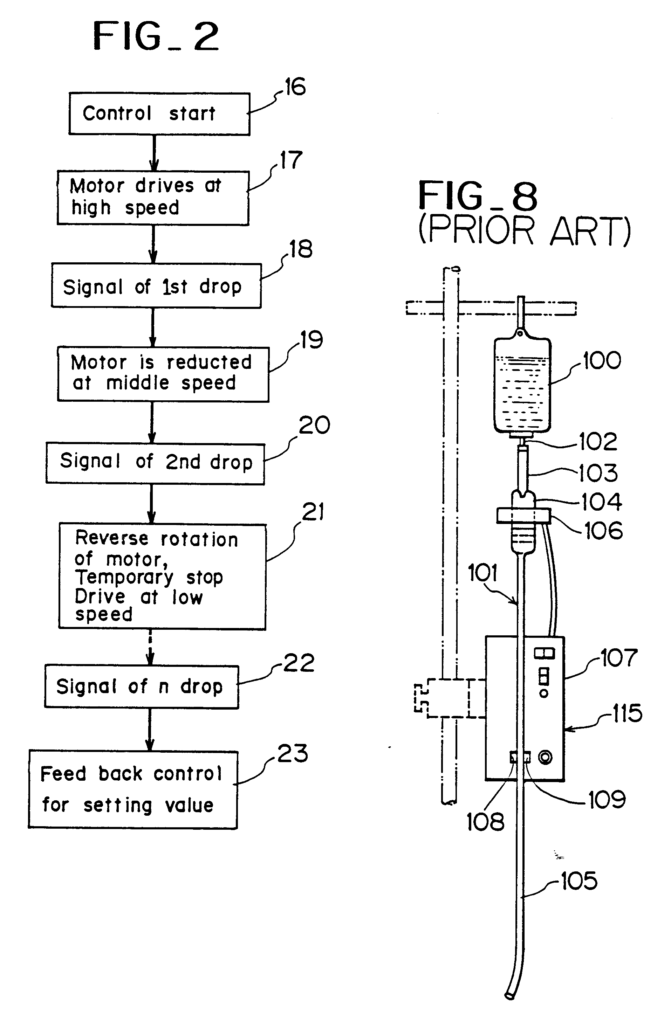

Fig. 2 is a flow chart of the liquid dripping control system using the microcomputer 14.

The motor of the cam drive unit 12 is reversed by the signal from the control start 16 of the device 1, and the clamping jaw 11 moves and presses the flexible tube 4 to the stationary clamping jaw 7, and the tube 4 is perfectly closed (in the following called "control starting point").

Then, the motor is rotated forward at high speed 17, and the clamping jaw 11 moves at the high speed 17 the flexible tube 4 in the opening direction, and a first drop in the dropping tube 5 is produced rapidly.

The drop detecting circuit 8 detects the first drop as a dripping signal 18, and the motor is reduced 19 to the middle speed so that the movable clamping jaw 11 is reduced in movement.

After detecting a dripping signal 20 of a second drop, the motor is reversed in a moment, and the clamping jaw 11 is slightly moved in a throttling direction of the tube 4, and the rotation of the motor is stopped temporarily, so that the movement of the clamping jaw 11 is stopped 21 in the following "drop starting point". This is because, since dripping of the medical liquid of the first drop is rapidly induced, an excessive opening of the tube 4 is slightly revised when the clamping jaw 11 is moved to the opening direction at the maximum speed. Taking into consideration the resiliency and recovery of the flexible tube, attention is paid to a responding ability in shaping at the held portion of the bendable tube 4 by moving of the clamping jaw 11.

When the dripping signal 22 of a n-th drop is detected, the motor is rotated at the low speed (shown by "21"), and while the clamping jaw 11 is moved at the low speed in the opening or throttling direction of the flexible tube 11, it is moved by a feedback control 23 to a predetermined value.

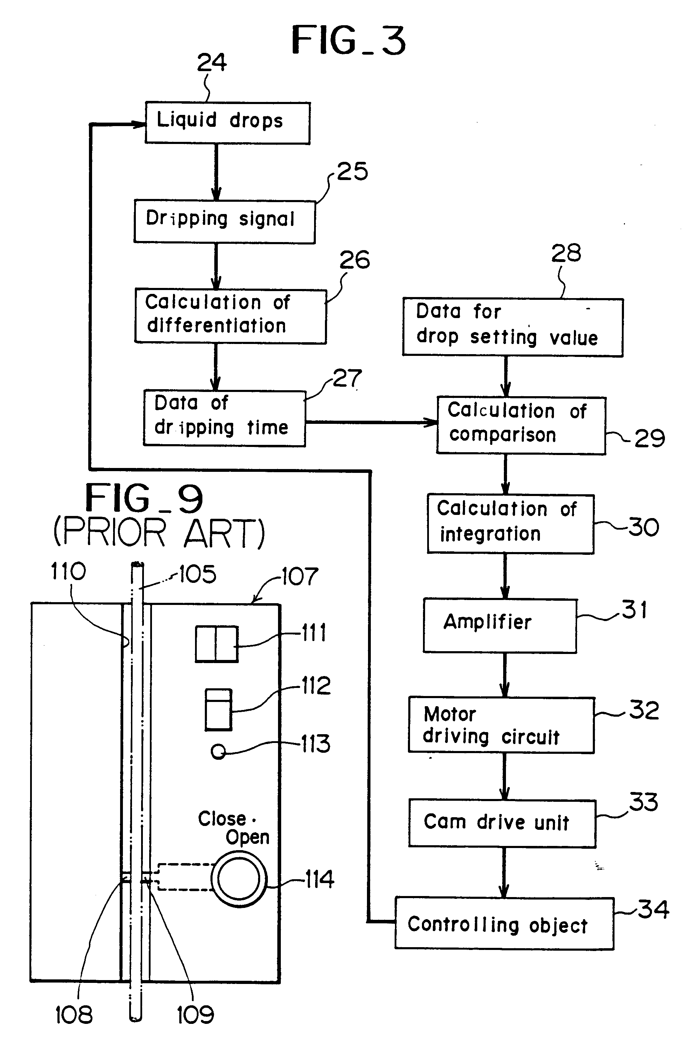

Fig. 3 shows a block diagram of the feedback control 23. A dripping signal 25 of the drop 24 of the n drop is input into a differentiation circuit 26, and after a dripping time data 27 is calculated, the signal 25 is fed into a comparison calculating circuit 29. This circuit 29 has been supplied with data 28 for a drop set value and after a comparison is made with the dripping time data 27, an output signal therefrom is input into the motor drive circuit 32 via the differentiation circuit 30 and an amplifier circuit 31. The input signal in the motor drive circuit 32 is inverted into an output signal for driving the motor and the rotation of the motor constituting the cam drive unit 12 is controlled. In accompany therewith, the calmping jaw 11 is moved via the mechanisms such as cams and gears, and an object 33 (the amount of throttling the tube) to be controlled is adjusted, so that a dripping interval of n + 1 drop is controlled. The drop of this n + 1 drop is a new dropping signal 25, and subsequently the dripping speed is controlled.

Fig. 4 is a graph showing changes of the dripping as time passes when the dripping of the liquid is controlled by the present device. This graph will be obvious in comparison with the flow chart of Fig. 2. The throttling amount is the maximum at the control starting point, and the dripping number is zero. T is time until the drop starting point from rapid induction of the dripping of the 1st drop in correspondence to 17 to 21 of Fig. 2. At T, the difference between the predetermined dripping rate and the actual dripping rate is controlled to be substantially zero, and the dripping rate is controlled not to be outside of a control range n'. n shows a control limiting scope by the present device.

If the dripping rate is abnormally decreased or increased, the movable clamping jaw 11 is returned to a cancelling point of the control start, that is, 0 point at the maximum throttling, and stops the dropping.

Fig. 5 is a graph showing relationship between the amount of the throttling of the tube and the dripping number or rate, where A, A', A" show the drop starting points, and B, B', B" are approximately around the predetermined points, and C, C', C" show the maximum dripping number points. In the same, the curve III (A - B - C) relates to the present invention, and the curve I (A' - B' - C') and the curve II (A" - B" - C") relate to the conventional examples where are used the tubes of different thicknesses and different materials. It is seen that the device of the present invention may control the dripping number within the determined control range, irrespectively of the type of the liquid sending sets.

Fig. 6 is a front view of the present control device, and Fig. 7 is a side view of the same.

The device is formed with a central groove 40 for attaching the lower flexible tube 4 of the liquid sending set, the groove 40 being formed with an intermediate groove portion 41 of large width. The groove portion 41 has the same depth as the tube attaching groove 40, and its width is as large as a finger would be adapted. In Figs. 6 and 7, the numeral 43 is a dripping number set switch, 44 is a switch of an electric source, 45 is an alarm lamp and 42 is a thumb for opening and closing the movable clamping jaw, as in the prior art.

A further reference will be made a sequential operation for attaching the flexible tube to the present device.

The thumb 42 is opened to make a clearance between the stationary clamping jaw 7 and the movable clamping jaw 11, and the tube 4 is stretched by the both hands and fitted to the groove 40, and both ends of the tube 4 are forced to the bottom of the groove 40. The tube 4 in the intermediate groove portion 41 is pushed by the finger, and the upper part and the lower part of the tube outside of the groove portion 41 are separately fitted into the attaching groove 40 in the length. The lower tube is held between the stationary clamping jaw 7 and the movable clamping jaw 11.

The intermediate groove portion 41 may be positioned in any part nearly around the both clamping jaw 7 and 11, for example, at the lower part of them.

The present invention is not limited to the above mentioned embodiment. The invention may be utilized to not only the medical liquid control but also dripping controls of the liquid to be used in the physical and chemical experimental fields.

## Claims

1. A device for controlling a liquid dripping rate of a medical liquid spending set which has a flexible tube (4) for leading said liquid, and a dripping tube (5) connected to said flexible tube (4), comprising

   a stationary clamping jaw (7) and a movable clamping jaw (11) for clamping said flexible tube (4),

   a device (6a,6b) for detecting dripping of said liquid into said dripping tube (5),

   control means (14) for controlling operation of a drive device (10,12) of said movable clamping jaw (11) in association with said drip detecting device (6a,6b),

   said control means (14) being provided with means for feedback controlling the dripping rate to be a determined rate while moving at a first speed said movable clamping jaw (11) in the direction of throttling or releasing said flexible tube (4),

   characterized in that said control means (14) further is provided with

   means for moving said movable clamping jaw (11) in a direction of throttling said flexible tube (4) by a signal for starting the control, and completely closing said flexible tube (4) by said stationary and said movable clamping jaws (7,11),

   means for moving at a second speed said drive device (10,12) of said movable clamping (11) in the direction of releasing said flexible tube (4), said second speed being higher than said first speed, so as to rapidly start dripping from the closed condition of said flexible tube (4),

   means for reducing the speed of moving said movable clamping jaw (11) after having detected a first drip, and

   means for slightly moving said movable clamping jaw (11) in the direction of throttling said flexible tube (4) after having detected a second drip.

2. The device as claimed in claim 1, further comprising a means for rapidly closing the tube (4) by the movable clamping jaw (11) when dripping rate is abnormal, and issuing an alarm output.

3. The device as claimed in claim 1 or 2, characterized in that said feedback control means is including

means for calculating a dripping signal from the dripping detecting device (6a,6b) by differentiation;

means for comparing liquid dripping time data and dripping predetermined data; and

means for moving the movable clamping jaw (11) to provide a liquid dripping of determined value in response to a signal from the comparison calculation so as to adjsut the amount of throttling of the flexible tube.

4. The device as claimed in claim 1, 2 or 3, comprising

a liquid dripping sensor (6a,6b) for detecting dripping of a liquid staying in said dripping tube (5), a liquid drip detecting circuit and a dripping number setting circuit;

a drive circuit to be connected to a drive unit of the movable clamping jaw (11);

a microcomputer connected to each of said circuits; and further comprising

means for causing the microcomputer to issue a high speed control signal and moving said movable clamping jaw (11) at said second speed to a direction of releasing the tube, so as to generate said first drip from zero drip;

means for giving said signal to the drive signal through the microcomputer, after having detected said second drip from the drip detecting circuit, and moving the movable clamping jaw (11) to a direction of slightly throttling the tube and stopping said movable clamping jaw (11); and

means for causing the microcomputer to issue a low speed control signal from a third drip to a n-th drip, and moving the movable clamping jaw (11) to a direction of releasing the tube at said first speed.

5. The device as claimed in one of the previous claims, characterized in that a groove (40) is formed in height of the device for holding said flexible tube (4), said groove extending in opposite directions from the clamping jaws (7,11), said groove being formed with an intermediate groove portion (41) of enlarged width.

**Revendications**

1. Dispositif pour contrôler un débit d'écoulement goutte-à-goutte d'un ensemble de distribution de liquide médical, qui comporte un tube flexible (4) pour amener ledit liquide, et un tube d'écoulement goutte-à-goutte (5) relié audit tube flexible (4), comprenant:

un mors stationnaire (7) et un mors mobile (11), pour serrer ledit tube flexible (4),

un dispositif (6a, 6b), servant à détecter l'écoulement goutte-à-goutte dudit liquide dans ledit tube d'écoulement goutte-à-goutte (5),

un moyen de contrôle (14) pour contrôler le fonctionnement d'un dispositif d'entraînement (10, 12) dudit mors mobile (11), en association avec ledit dispositif (6a, 6b) de détection d'écoulement goutte-à-goutte,

ledit moyen de contrôle (14) étant pourvu d'un moyen servant à contrôler de façon rétroactive le débit d'écoulement goutte-à-goutte, pour qu'il soit égal à un débit déterminé, tout en déplaçant ledit mors mobile (11) à une première vitesse, dans le sens de l'étranglement ou du desserrage dudit tube flexible (4),

caractérisé en ce que ledit moyen de contrôle (14) est en outre pourvu:

d'un moyen de déplacement dudit mors mobile (11) dans le sens de l'étranglement dudit tube flexible (4), par un signal de démarrage de la procédure de contrôle, et

fermant complètement ledit tube flexible (4) grâce auxdits mors stationnaire et mobile (7, 11),

d'un moyen de déplacement dudit dispositif d'entraînement (10, 12) dudit mors mobile (11) à une seconde vitesse, dans le sens du desserrage dudit tube flexible (4), ladite seconde vitesse étant supérieure à ladite première vitesse, de façon à démarrer rapidement l'écoulement goutte-à-goutte en partant de l'état fermé dudit tube flexible (4),

d'un moyen de réduction de la vitesse de déplacement dudit mors mobile (11) , après avoir détecté un premier écoulement goutte-à-goutte, et

d'un moyen de léger déplacement dudit mors mobile (11) dans le sens de l'étranglement dudit tube flexible (4), après avoir détecté un second écoulement goutte-à-goutte.

2. Dispositif selon la revendication 1, comprenant en outre un moyen de fermeture rapide du tube (4) par le mors mobile (11), lorsque le débit d'écoulement goutte-à-goutte est anormal, et d'émission d'un signal de sortie d'alarme.

3. Dispositif selon la revendication 1 ou 2, caractérisé en ce que ledit moyen de contrôle à réaction comprend:

un moyen de calcul par différentiation d'un signal d'écoulement goutte-à-goutte provenant du dispositif de détection d'écoulement goutte-à-goutte (6a, 6b);

un moyen de comparaison d'une donnée temporelle et d'une donnée prédéterminée d'écoulement goutte-à-goutte; et

un moyen de déplacement du mors mobile (11), pour fournir un écoulement goutte-à-goutte du liquide d'une valeur déterminée, en réponse à un signal provenant du calcul comparatif, de façon à ajuster la valeur de l'étranglement du tube flexible.

4. Dispositif selon la revendication 1, 2 ou 3, comprenant:

un détecteur d'écoulement goutte-à-goutte de liquide (6a, 6b), servant à détecter l'écoulement goutte-à-goutte d'un liquide contenu dans ledit tube d'écoulement goutte-à-goutte (5), un circuit de détection de gouttes de liquide, et un circuit de fixation du nombre de gouttes;

un circuit d'entraînement à relier à une unité d'entraînement du mors mobile (11);

un micro-ordinateur relié à chacun desdits circuits;

et comprenant en outre:

un moyen pour provoquer l'émission par le micro-ordinateur d'un signal de contrôle à grande vitesse, et le déplacement dudit mors mobile (11) à ladite seconde vitesse, dans le sens du desserrage du tube, de façon à produire ledit premier écoulement goutte-à-goutte en partant d'un écoulement goutte-à-goutte nul;

un moyen pour transmettre ledit signal au signal d'entraînement passant dans le micro-ordinateur, après avoir détecté ledit second écoulement goutte-à-goutte par le circuit de détection de gouttes, et déplacer le mors mobile (11) dans le sens d'un léger étranglement du tube, et stopper ledit mors mobile (11); et

un moyen pour provoquer l'émission par le micro-ordinateur d'un signal de contrôle à faible vitesse, à partir d'un troisième écoulement goutte-à-goutte jusqu'à un n$^{ième}$ écoulement goutte-à-goutte, et le déplacement du mors mobile (11) dans le sens du desserrage du tube, à ladite première vitesse.

5. Dispositif selon l'une des revendications précédentes, caractérisé en ce qu'un canal (40) est formé en hauteur dans le dispositif, afin de maintenir ledit tube flexible (4), ledit canal s'étendant dans des directions opposées en partant des mors (7, 11), ledit canal étant formé avec une partie de canal intermédiaire (41) de plus grande largeur.

**Patentansprüche**

1. Vorrichtung zur Regelung einer Flüssigkeitstropfrate eines medizinischen Flüssigkeitsabgabesatzes, der eine flexible Röhre (4) zum Führen der Flüssigkeit und eine mit der flexiblen Röhre (4) verbundene Tropfröhre (5) aufweist, enthaltend
einen stationären Klemmbacken (7) und beweglichen Klemmbacken (11) zum Klemmen der flexiblen Röhre (4),
eine Vorrichtung (6a,6b) zum Erfassen eines Tropfens der Flüssigkeit in die Tropfröhre (5),
eine Regeleinrichtung (14) zur Regelung des Betriebs einer Antriebsvorrichtung (10,12) des beweglichen Klemmbackens (11) in Verbindung mit der Tropferfassungsvorrichtung (6a,6b),
wobei die Regeleinrichtung (14) versehen ist mit einer Einrichtung zur Rückkopplungsregelung der Tropfrate, so daß sie eine vorgegebene Rate ist, wobei der Klemmbacken (11) mit einer ersten Geschwindigkeit in Richtung einer Drosselung oder eines Freigebens der flexiblen Röhre (4) bewegt wird,
**dadurch gekennzeichnet,** daß die Regeleinrichtung (14) weiterhin versehen ist mit
einer Einrichtung zum Bewegen des beweglichen Klemmbackens (11) in einer Richtung einer Drosselung der flexiblen Röhre (4) durch ein Signal zum Starten der Regelung und vollständigen Schließen der flexiblen Röhre (4) durch den stationären und den beweglichen Klemmbacken (7,11),
einer Einrichtung zum Bewegen der Antriebsvorrichtung (10,12) des beweglichen Klemmbackens (11) in der Richtung eines Freigebens der flexiblen Röhre (4) mit einer zweiten Geschwindigkeit, wobei die zweite Geschwindigkeit höher ist als die erste Geschwindigkeit, so daß vom geschlossenen Zustand der flexiblen Röhre (4) das Tropfen schnell beginnt,

einer Einrichtung zum Vermindern der Geschwindigkeit des Bewegens des beweglichen Klemmbackens (11) nachdem ein erster Tropfen erfaßt worden ist, und

einer Einrichtung zum geringfügigen Bewegen des beweglichen Klemmbackens (11) in der Richtung eines Drosselns der flexiblen Röhre (4) nachdem ein zweiter Tropfen erfaßt worden ist.

2. Vorrichtung nach Anspruch 1, weiterhin enthaltend eine Einrichtung zum schnellen Schließen der Röhre (4) mittels des beweglichen Klemmbackens (11), wenn die Tropfrate abnorm ist, und Ausgeben eines Alarmausgangssignals.

3. Vorrichtung nach Anspruch 1 oder 2, **dadurch gekennzeichnet,** daß die Rückkopplungsregeleinrichtung enthält

eine Einrichtung zum Berechnen eines Tropfsignals von der Tropferfassungseinrichtung (6a,6b) durch Differentiation;

eine Einrichtung zum Vergleichen von Flüssigkeitstropfzeitdaten und vorgegebenen Tropfdaten; und

eine Einrichtung zum Bewegen des beweglichen Klemmbackens (11), um in Ansprache auf ein Signal von der Vergleichsberechnung für ein Tropfen der Flüssigkeit mit einem vorgegebenen Wert zu sorgen, so daß der Betrag der Drosselung der flexiblen Röhre eingestellt wird.

4. Vorrichtung nach Anspruch 1, 2 oder 3, enthaltend

einen Flüssigkeitstropfsensor (6a,6b) zum Erfassen des Tropfens einer in der Tropfröhre (5) stehenden Flüssgikeit, eine Flüssigkeitstropferfassungsschaltung und eine Tropfenanzahleinstellschaltung;

eine mit einer Antriebsvorrichtung des beweglichen Klemmbackens (11) zu verbindende Treiberschaltung;

einen mit jeder der Schaltungen verbundenen Mikrocomputer;

und weiterhin enthaltend

eine Einrichtung, die bewirkt, daß der Mikrocomputer ein Regelsignal für hohe Geschwindigkeit ausgibt und zum Bewegen des beweglichen Klemmbackens (11) mit der zweiten Geschwindigkeit in einer Richtung eines Freigebens der Röhre, so daß von null Tropfen der erste Tropfen erzeugt wird;

eine Einrichtung zum Abgeben des Signals über den Mikrocomputer an die Antriebsvorrichtung, nachdem der zweite Tropfen von der Tropfenerfassungsschaltung erfaßt worden ist, und Bewegen des beweglichen Klemmbackens (11) in einer Richtung eines geringfügigen Drosselns der Röhre und Anhalten des beweglichen Klemmbackens (11); und

eine Einrichtung, die bewirkt, daß der Mikrocomputer von einem dritten Tropfen bis zu einem n-ten Tropfen ein Regelsignal für niedrige Geschwindigkeit abgibt, und zum Bewegen des beweglichen Klemmbackens (11) in einer Richtung eines Freigebens der Röhre mit der ersten Geschwindigkeit.

5. Vorrichtung nach einem der vorhergehenden Ansprüche, **dadurch gekennzeichnet,** daß in der Höhe der Vorrichtung eine Ausnehmung (40) zum Halten der flexiblen Röhre (4) ausgebildet ist, sich die Ausnehmung von den Klemmbacken (7,11) in entgegengesetzten Richtungen erstreckt, und die Ausnehmung mit einem Ausnehmungszwischenbereich (41) vergrößerter Breite versehen ist.

# FIG_1

| | |
|---|---|
| Drop detecting circuit | Dripping number setter |

Cam drive unit

Drive unit

Close · Open

Microcomputer

Alarm output

# FIG_6

Close · Open

# FIG_7

# FIG_2

Control start — 16

Motor drives at high speed — 17

Signal of 1st drop — 18

Motor is reduced at middle speed — 19

Signal of 2nd drop — 20

Reverse rotation of motor, Temporary stop Drive at low speed — 21

Signal of n drop — 22

Feed back control for setting value — 23

# FIG_8
## (PRIOR ART)

10

# FIG_3

24

Liquid drops

25

Dripping signal

26

Calculation of differentiation

27

Data of dripping time

28

Data for drop setting value

Calculation of comparison 29

Calculation of integration 30

Amplifier 31

Motor driving circuit 32

Cam drive unit 33

Controlling object 34

# FIG_9
## (PRIOR ART)

110 105 107

111

112

113

Close · Open

114

108 109

# FIG_4

Setting value

Time/Minute

T

Time

n'

n

# FIG_5

Maximum

Dripping number

C' C C"

I III II

B' B B"

A' A A"

Throttling number of tube

Small

# FIG_10
## (PRIOR ART)

Maximum

Dripping number

C' C"

B' B"

A' A"

Throttling number of tube

Small